# EUROPEAN PATENT SPECIFICATION

(11) **EP 2 501 367 B1**
(45) Date of publication and mention of the grant of the patent: **21.01.2015**
(21) Application number: 10784450.8
(22) Date of filing: 17.11.2010
(51) Int. Cl.: A61K 9/19, A61K 47/18, A61K 38/27, A61K 38/30

(54) **Formulation for HGH and RHIGF-1 combination**
Formulierung für HGH- und RHIGF-1-Kombination
Préparation pour combinaison d'hormones de croissance humaine (HGH) et de RHIGF-1

(30) Priority: 17.11.2009 US 261859 P
(43) Date of publication of application: 26.09.2012
(73) Proprietor: IPSEN PHARMA S.A.S., 92100 Boulogne-Billancourt (FR)
(72) Inventor: GOPINATH, Enona, Redwood City California 94062 (US); PARK, Susan, San Francisco California 94112 (US); ARAKAWA, Tsutomu, Thousand Oaks CA 91360 (US); RICHARD, Joël, F-78490 Méré (FR); FAIS, Fabio, F-28100 Dreux (FR)
(74) Representative: Audonnet, Nathalie
(86) International application number: PCT/EP2010/006996
(87) International publication number: WO 2011/060922

(56) References cited:
- EP-A1- 1 369 125
- WO-A1-01/03741
- WO-A1-96/21460
- US-A- 5 763 394
- US-B1- 6 767 892

## Description

The present invention relates to pharmaceutical compositions. More particularly, the invention relates to formulations of growth hormone (GH) and insulin-like growth factor (IGF-1) combination compositions. These combination compositions provide stable liquid pharmaceutical compositions without the formation of visible insoluble aggregates at a desirable pH.

The present invention further provides a formulation for insulin-like growth factor 1 (IGF-1) and growth hormone (GH), wherein proteins may be formulated together in an injectable form, or formulated separately and mixed into a unit dosable injectable form prior to administration.

Insulin-like growth hormone belongs to the family of polypeptides known as somatomedins and is a polypeptide naturally occurring in human body fluids. Most tissues and especially the liver produces IGF-1 together with specific IGF-binding proteins. IGF-1 stimulates growth and division of a variety of cell types, particularly during development, thus processes such as skeletal growth and cell replication are affected by IGF-1 level. These molecules are under the control of growth hormone (GH).

IGF-1 is the primary protein hormone mediating the growth promoting effects of GH on bone. IGF-1 is produced in response to GH and then induces subsequent cellular responses, including cellular responses in bone. IGF-1 is composed of 70 amino acids in a single chain with three intramolecular disulfide bridges. IGF-1 has a molecular weight of 7649 daltons and is produced primarily by the liver as an endocrine hormone as well as in target tissues in a paracrine/autocrine fashion. IGF-1 has been manufactured recombinantly (rhIGF-1) on a large scale using both yeast and E. coli.

Growth hormone or human growth hormone (hGH) is a single-chain polypeptide consisting of 191 amino acids. Disulfide bonds link positions 53 and 165 and 182 and 189. Human GH is a potent anabolic agent. Among its most striking effects in hypopituitary (GH deficient) subjects is accelerated linear growth of bone growth plate cartilage resulting in increased stature.

The advantageous and synergic effect of the combination of both proteins is described in the international patent application WO9118621. Co-administration of IGF-1 and GH to a mammal gives rise to enhanced growth over the growth achieved using either IGF-1 or GH alone. The enhancement is equal to the sum of the growth observed when IGF-1 is administered and the growth observed when GH is administered.

Methods and compositions for increasing the growth rate are also disclosed in the international patent application WO 2006/130769. The study related essentially to a method of treatment and the results focused on the patient reaction. Pharmaceutical compositions are described and particularly a mixture of IGF-1 and GH formulated in mannitol, glycine and/or phosphate at pH 7.4. If the mixture is to be stored, it is formulated in a buffer such as citrate at a pH of about 6, with a surfactant that increases the solubility of the GH at this pH such as polysorbate 20 or poloxamer 188. It also describes the possibility of adding an inorganic salt and a stabilizer. No non-aggregating agent is used in the formulations disclosed in WO 2006/130769.

A problem frequently occurring when combining two proteins in a solution is the formation of complexes by protein-protein interactions. Such formation of complexes is particularly influenced by change in concentration, temperature, pH and buffer of the protein-containing solutions. The protein complexes may then form insoluble aggregates causing loss of potency and activity of the proteins.

Furthermore, in pharmaceutical formulations, the dosage of therapeutic protein is important and must be kept within controlled ranges over an extended period of time. The use of solubilizing agents is often required to obtain and maintain the right concentration of protein in solution and particularly to solubilize high amounts of proteins. US patent 6,767,892 disclosed pharmaceutical compositions of IGF-1 and analogues thereof containing solubilizing compounds such as arginine, N-acetyl arginine or guanidine hydrochloride IGF-1. Compositions were tested, comparative data were provided with increased IGF-1 solubility at pH greater than 5.0 and at refrigerated temperatures. However this document does not disclose compositions comprising IGF-1 combined with further therapeutic proteins.

It is an object of the invention to prepare liquid formulations containing both IGF-1 and growth hormone (GH), which are stable at 4 °C for at least 30 days, with no significant aggregation as evidenced by visual clarity of the solution. A process for the preparation of a liquid formulation containing both IGF-1 and GH is a further object of the invention. Description of figures:

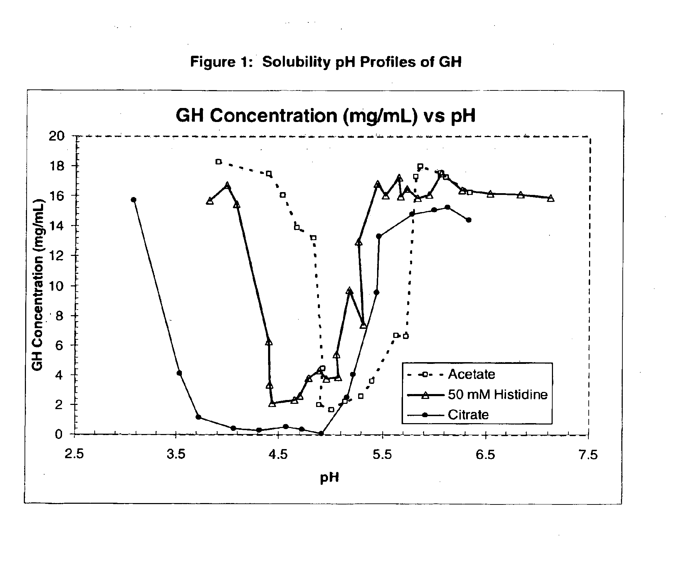

- Figure 1:: shows overlaid sedimentation velocity profiles obtained by analytical ultracentrifugation of a IGF-1 solution, GH solutions, and a 1:1 mixture of the two solutions. The first set of profiles (Figure 1) was obtained with the proteins formulated in a 25 mM citrate buffer at pH 6, and shows evidence of substantial association between the proteins.

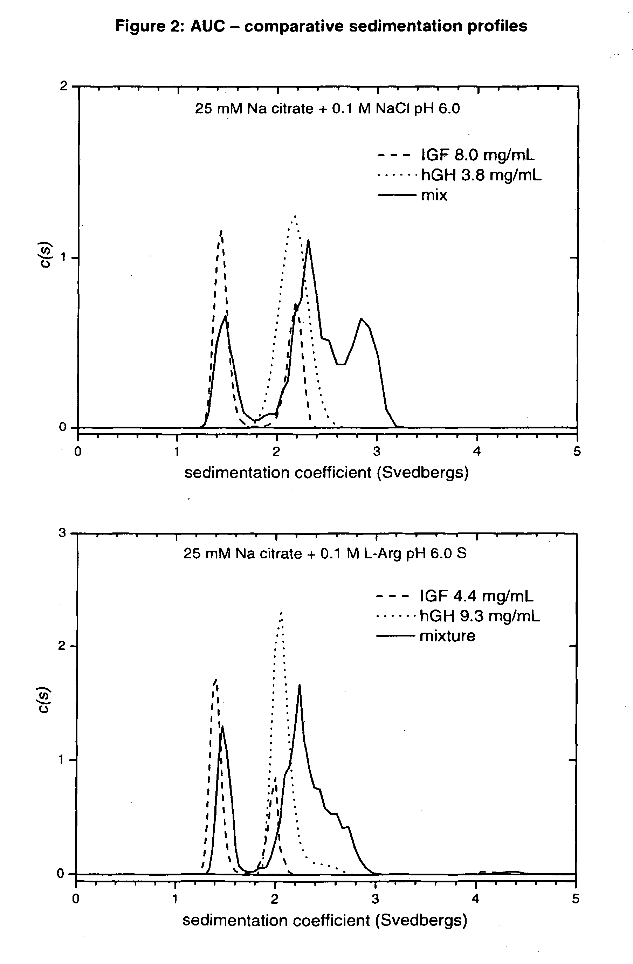

- Figure 2:: shows sedimentation profiles of solutions including 100 mM argininium ion (arginine). The profiles show that the presence of arginine produces changes indicative of a reduced amount of high-molecular weight aggregates in the solutions.

The following definitions are set forth to illustrate and define the meaning and scope of the various terms used to describe the invention herein.

According to the present invention the term "non-aggregating agent" relates to compounds which prevent or reduce formation of insoluble protein aggregates, when proteins are put in a solution.

The term "IGF-1" refers to insulin-like growth factor-1 from any species including but not limited to bovine, ovine, porcine, avian and preferably human in native-sequence or in variant form and from any source, whether natural synthetic or recombinant.

Preferably, IGF-1 is recombinantly produced as e.g. described in US 6,331,414. More preferably, IGF-1 is the active pharmaceutical ingredient in the product commercially marketed as INCRELEX^{™}.

The term "rhIGF-1" refers to recombinant human IGF-1.

The term "GH" refers to growth hormone from any species including but not limited to bovine, ovine, porcine, avian and preferably human in native-sequence or in variant form and from any source, whether natural synthetic or recombinant.

The terms "human growth hormone" and "hGH" relate to human growth hormone produced by methods including natural source extraction and purification, and by recombinant cell culture systems for instance as disclosed in the scientific publication *"*Direct expression in Escherichia coli of a DNA sequence coding for human growth hormone" Goeddel & al, Nature Vol. 281, October 1979*.* The sequence of hGH is set forth, for example in Hormone Drugs, Gueriguian et al., USP convention, Rockville, MD (1982). The terms also cover biologically active human hormone equivalents, e.g., including one or more different amino acid(s) in the overall sequence. Furthermore, the terms as used in this application are intended to cover substitution, deletion and insertion amino acid variants of hGH, i.e., analogs and/or homologs of hGH or hGHs with posttranslational modifications. Two species are often used: the 191 amino acid native species (Somatropin) and the 192 amino acid N-terminal methionine species, both commonly obtained recombinantly.

It is preferred to use methionyl human growth hormone (met-hGH) produced in E.coli, which is sold under the trademark PROTROPIN^{®} by Genentech, Inc. and is identical to the natural polypeptide, with the exception of the presence of an N-terminal methionine residue. Also preferred is the recombinant hGH available from Genentech, Inc. under the trademark NUTROPIN^{®}. More preferred is recombinant rhGH liquid for injection available from Genentech, Inc. under the trademark NUTROPIN AQ^{®}.

The term "buffer" as used herein denotes a pharmaceutically acceptable buffer which preferably confers a pH of 5-6.5. Suitable buffers comprise but are not limited to acetate buffers, citrate buffers, phosphate buffers, succinate buffers and amino acid buffers such as histidine buffers and all salts thereof.

The term "preservative" as used herein means a pharmaceutically acceptable substance to prevent decomposition by microbial growth or by undesirable chemical change.

The terms "surfactant" as used herein means a pharmaceutically acceptable substance to allow dispersion or suspension, by reducing the surface tension of the solvent (such as water) or the interfacial tension between two non miscible liquids. Suitable surfactants are for instance non ionic surfactants such as polysorbates or poloxamers.

The term "bulking agent" as used herein means a pharmaceutically acceptable substance used to increase the amounts of solids and are for instance sucrose, trehalose and mannitol, but not limited to those listed.

The term "tonicity modifer" refers to an isotonic modifier or osmotic adjuster or osmolyte that provides osmolality to the buffer solution. Osmolality refers to the total osmotic activity contributed by ions and nonionized molecules to a solution which includes inorganic salts such as sodium chloride and potassium chloride, polyethylene glycols (PEGs), polypropylene, glycol, glycine, glycerol.

The term "lyophilised" as used herein refers to a formulation that has undergone a process known in the art as freeze-drying, involving freezing the formulation and subsequently removing the ice from the frozen content.

The term "amino acid" as used herein denotes an amino acid (a free amino acid, i.e. not an amino acid in a peptide or protein sequence). An amino acid, as used herein, comprises but is not limited to arginine, glycine, lysine, histidine, glutamic acid, aspargic acid, isoleucine, leucine, alanine, phenylalanine, tryptophan, serine, methionine and proline, for instance.

The term "IRF" or "immediate release formulation" refers to a drug composition or mixture of drug compositions, preferably is liquid form, in which there is no carrier that regulates the bioavailability of the drug's active substance to tissues at the site of drug administration in the patient's body.

The term "non-aggregating agent" as used herein refers to a product which prevents the interaction of proteins to form complexes and/or aggregates when they are mixed together in a solution.

In accordance with the present invention, the pharmaceutical composition comprises rhIGF-1 and rhGH and
- a non-aggregating agent;
- a buffer;
- a non-ionic surfactant;
- a preservative; and
- a tonicity modifier or bulking agent,
wherein the non-aggregating agent is arginine present in the composition in a concentration ranging from 80 mM to 200 mM,
the buffer is selected from histidine or citrate at a concentration ranging from 1 to 50 mM, and the tonicity modifier is sodium chloride at a concentration of 0 to 150 mM.

It is a characteristic of the pharmaceutical composition of the invention that the two active ingredients IGF-1 and GH are present in a single formulation. A "single formulation", as used herein, is also referred to as a "co-formulation" or a "co-mix". The terms co-formulation or co-mix are used interchangeably herein.

Preferably, the two active ingredients are human IGF-1 and GH, also called hIGF-1 and hGH herein. It is further preferred that both active ingredients are produced by recombinant means.

In a preferred embodiment, the pharmaceutical composition of the invention is a liquid composition. It is further preferred that it is a multi-dose composition. In the embodiment of a multi-dose composition, a preservative is preferably present.

In a further aspect, the invention relates to processes for the preparation of a pharmaceutical composition comprising IGF-1 and GH. One process according to the invention for the preparation of a pharmaceutical composition may be carried out as follows:
a) Preparing a hGH solution in a buffer at a pH between 5 and 6.5 comprising the non-aggregating agent, the tonicity modifier or bulking agent;
b) Preparing a solution of IGF-1 by dialysing an IGF-1 preparation into the buffer used in step (a) comprising said non-aggregating agent and said tonicity modifier or bulking agent;
c) Adding the surfactant and the preservative to both stock solutions; and
d) Mixing together the solutions of hGH and IGF-1.

In embodiments of this process, in step (a), lyophilized hGH is dissolved in a buffer, or liquid hGH (e.g. approximately 20 mg/ml solution in bicarbonate buffer) is buffer exchanged into another buffer, preferably citrate, succinate or histidine buffer at a convenient pH, preferably between about 5 and 6.5, the buffer containing the non-aggregating agent at a concentration range of between 80 to 200 mM, preferably in the range of between about 100 mM and about 150 mM. Optionally, at least one solution prepared in any of steps (a), (b), (c) or (d) comprises a preservative, preferably phenol or benzyl alcohol.

The term "about", in the context of amounts of ingredients presented herein, means that the amount can vary by less than ±20% or less than ±15% or less than ±10% or less than ±5%.

In step (b), lyophilized IGF-1 is dissolved into a buffer, or liquid IGF-1 (e.g. approximately 20-35 mg/ml solution in citrate buffer) is buffer exchanged into another buffer, preferably citrate, succinate or histidine at a convenient pH, preferably between about 5 and 6.5, the buffer containing the non-aggregating agent at a concentration range from about 80 mM to about 200 mM.

The two independently prepared solutions are then mixed together.

An alternative process for the preparation of a pharmaceutical composition is also encompassed by the invention.

In accordance with the present invention, the alternative process for the preparation of a pharmaceutical composition of the invention comprises:
a) Preparing a solution I by admixing the buffer, preferably histidine buffer, the non-aggregating agent, the surfactant, preferably polysorbate 20, the preservative, preferably benzyl alcohol, and optionally adjusting the volume with water, the solution I having or being adjusted to a pH of about 5.8;
b) Preparing a solution of IGF-1, in the buffer and non-aggregating agent that are used in step (a), to obtain a solution II;
c) Adding solution II to solution I to obtain a solution III;
d) Preparing a solution IV by admixing the buffer, preferably histidine, the non-aggregating agent, the surfactant, preferably polysorbate 20, the preservative, preferably benzyl alcohol, and optionally adjusting the volume with water, the solution IV having or being adjusted to a pH of about 5.8;
e) Preparing a solution of GH in the buffer and non-aggregating agent that are used in step (d), the GH optionally comprising sodium bicarbonate buffer, in order to obtain a solution V;
f) Adding solution V to solution IV to obtain a solution VI;
g) Optionally, independently filtering solutions III and VI;
h) Mixing filtered solutions III and VI at a ratio of IGF-1:GH (w/w) between 1:1 and 7:1 (w/w), preferably 1.1:1 (w/w), 3.3:1 (w/w) and 6.6:1, to obtain a solution VII; and
i) Optionally, filtering solution VII.

Steps (b) and (e) can e.g. be carried out by diafiltration of a solution comprising IGF-1 or GH into the appropriate buffer and non-aggregating agent or any other suitable solution in order to obtain solutions II and IV.

In an embodiment, solution I and solution IV are identical. In that embodiment, step (d) is obsolete, i.e. solution IV is not prepared and solution V is simply mixed with solution I to obtain solution VI.

In an embodiment, solutions II and IV may comprise a bulking agent such as e.g. sucrose or mannitol.

In an embodiment, a liquid GH drug substance (i.e. a solution comprising GH, preferably hGH and more preferably rhGH) is directly mixed with solution IV, without any prior buffer exchange or diafiltration into the buffer and non-aggregating agent according to step (e), i.e. without performing step (e) as described above.

Hence, in this embodiment, the process comprises the following steps:
a) Preparing a solution I by admixing the buffer, preferably histidine buffer, the non-aggregating agent, the surfactant, preferably polysorbate 20, the preservative, preferably benzyl alcohol, and optionally adjusting the volume with water, the solution I having or being adjusted to a pH of about 5.8;
b) Preparing a solution of IGF-1, in the buffer and non-aggregating agent that are used in step (a), to obtain a solution II;
c) Adding solution II to solution I to obtain a solution III;
d) Preparing a solution IV by admixing the buffer, preferably histidine, the non-aggregating agent, the surfactant, preferably polysorbate 20, the preservative, preferably benzyl alcohol, and optionally adjusting the volume with water, the solution IV having or being adjusted to a pH of about 5.8;
e) Adding a GH drug substance, optionally comprising sodium bicarbonate buffer, to solution IV to obtain a solution VI;
f) Optionally, independently filtering solutions III and VI;
g) Mixing filtered solutions III and VI at a ratio of IGF-1:GH (w/w) between 1:1 and 7:1 (w/w), preferably 1.1:1 (w/w), 3.3:1 (w/w) and 6.6:1, to obtain a solution VII; and
h) Optionally, filtering solution VII.

In an embodiment of this variant process, solution I and solution IV are identical. In that embodiment, step (d) is obsolete, i.e. solution IV is not prepared, and the GH drug substance is simply mixed with solution I to obtain solution VI.

Preferably, the liquid hGH drug substance is approximately 20 mg/ml hGH solution in bicarbonate buffer of a concentration of about 6-10 mM, preferably 7.5 mM, and is diluted without preliminary diafiltration into a buffer, preferably citrate, succinate or histidine at a convenient pH, preferably between about 5 and 6.2 and optionally containing the non-aggregating agent at a concentration range from about 80 to 200 mM, preferably from about 100 mM or about 150 mM.

In another embodiment, a liquid IGF-1 (e.g. approximately 20-35 mg/ml solution in 200 mM citrate buffer) is buffer exchanged into another buffer, preferably citrate, succinate or histidine buffer at a convenient pH, preferably between about 5 and 6.5 and optionally containing the non-aggregating agent at a concentration range of about 80 to about 200 mM, preferably about 100 mM to about 150 mM. The two independently prepared solutions are then mixed together.

The filtration can be carried out by any suitable means, e.g. cellulose-based filters or PES (polyethersulfone) filters. In a preferred embodiment, filtrations of all solutions (before and after mixing the solutions) may be made by means of 0.22 micrometer filters of low affinity for proteins, such as e.g. polyvinylidene fluoride (PVDF) filters. The membranes of the filters preferably have molecular weight limits of about 5 kDa or about 3 kDa.

Advantageously, the pharmaceutical compositions of the invention are stable for at least one 1 month, 3 months, 6 months, 9 months, a year or up to 2 years.

In a further aspect, the present invention encompasses the use of arginine as a non-aggregating agent in a liquid pharmaceutical composition comprising IGF-1 and GH, preferably hIGF-1 and hGH, more preferably rhIGF-1 and rhGH, wherein the concentration of arginine ranges from about 80 mM to about 200 mM, i.e. is e.g. about 80, about 90 mM, about 100 mM, about 110mM, about 120 mM, about 130 mM, about 140 mM, about 150 mM, about 160 mM, about 170 mM, about 180 mM, about 190 mM or 200 mM.

It has been found that inclusion of an amino acid in the pharmaceutical composition allows mixtures of IGF-1 and GH to be formulated together in a clear solution formulation, with no loss of visual clarity in the mixture during subsequent refrigeration at 2 to 8 °C for at least 30 days, preferably for at least 6 months, more preferably for at least 12 months.

In a preferred embodiment of the invention, the formulation is stable upon storage at a temperature of -20 °C, or between 2°C and 8°C, for at least 18 months.

In one embodiment the invention encompasses a stable, co-miscible formulation of the active ingredients human Insulin-like growth factor 1 (hIGF-1) and human growth hormone (hGH). In a preferred embodiment, the active ingredients are produced by recombinant means and designated rhIGF-1 and rhGH.

The formulations comprise rhIGF-1 and rhGH, a non-aggregating agent, and a buffer. The formulations may contain a surfactant, preferably a non-ionic surfactant, optionally a preservative, and optionally a tonicity modifier And/or bulking agent.

The amino acid which allows mixtures of IGF-1 and GH to be formulated together in a clear solution formulation is arginine (for instance as argininium ion).

Preferably, the amino acid which acts as a non-aggregating agent is added separately to each solution before mixing together in a clear solution formulation. More preferably the final concentration of the non-aggregating agent in the clear solution is present at a concentration range of about 80 mM to about 200 mM or at a concentration range of about 100 mM to about 180 mM or at a concentration range of about 120 to about 160 mM or at a concentration of about 150 mM.

The pH is adjusted to a value ranging from about 5 to about 7, preferably from about 5.5 to about 6.5, more preferably from about 5.8 to 6.2. In the context of pH values, the term "about" means that the pH value can vary by ± 0.2 or ± 0.1. The pH of a solution can be adjusted by any suitable means, such as e.g. adding of an appropriate amount of an acidic solution such as e.g. citrate or, preferably, HCl.

The pH to be used in accordance with the present invention can be e.g. 5.1, 5.2, 5.3, 5.4, 5.5, 5.6, 5.7, 5.8, 5.9, 6.0, 6.1, 6.2, 6.3, 6.4, 6.5, preferably is 5.8, 6.2 or about 6.5.

In addition embodiment, the osmolyte or tonicity modifier is an inorganic salt. The inorganic salt is sodium chloride, present in the composition at a concentration of 0 to 150 mM, preferably in a concentration of 1 to 50 mM.

In addition, the preservative may be selected form the list: phenol, benzyl alcohol, m-cresol, chlorobutanol. Preferred preservatives are phenol or benzyl alcohol. The preservative may be present in the composition at a concentration of 0.1 to 5 % (w/w) preferably 0.2 to 2 % (w/w) or still preferably 1%.

The surfactant of the composition disclosed in the present invention is e.g. selected from the list: polysorbate (Tween) or a poloxamer such as polysorbate 80, polysorbate 20 or poloxamer 188. The surfactant is non ionic, more preferably is a polysorbate (Tween) such as polysorbate 80, polysorbate 20 or a poloxamer such as poloxamer 188, more preferably polysorbate 20 or poloxamer 188 in a concentration range from about 0.01 to 3 % (w/w) preferably from about 0.03 to 0.50 % (w/w) and more preferably of about 0.2% (w/w).

In addition, the buffer may be selected from suitable pharmaceutically acceptable buffers which confer a pH of 5 to 6.5 such as sodium citrate or histidine or both; preferably acetate buffers, citrate buffers, phosphate buffers amino acid such as histidine and all salts thereof. The buffer is selected from citrate or histidine. The buffer is present in the final composition at a concentration between 1 to 50 mM and more preferably from 10 mM to 20 mM.

In accordance with the invention the amounts of IGF-1 and GH are about 2 to 40 mg/ml (IGF-1) and about 1 to12 mg/ml (hGH) respectively, preferred amounts are about 5 to 20 mg/ml (IGF-1) and about 2 to 8 mg/ml (hGH). Further preferred amounts are about 10 mg/ml of IGF-1 and about 3 mg/ml of hGH, or about 13.2 mg/ml of IGF-1 and 2 mg/ml of GH.

The weight ratio of IGF-1:GH (w/w) ranges preferably from 1:1 to 9:1, or alternatively from 1:9 to 1:1. More preferably the weight ratio of IGF-1:GH (w/w) is selected from the list: 9:1 (w/w); 6:1 (w/w); 3:1 (w/w); 2:1; 3:7 (w/w); 1:1 (w/w); 1:2 (w/w); 1:5 (w/w); 7:3 (w/w); 9:1 (w/w).

More preferred weight ratios of IGF-1:GH (w/w) are selected from 1.1:1, 2.2:1, 3.3:1 and 6.6:1. In an embodiment, the composition comprises a combination of rhIGF-1 and rhGH in a concentration of about 10 to 30 mg/ml (IGF-1) and about 1 to 12 mg/ml (rhGH) respectively and a weight ratio of IGF-1:GH of between about 9:1 and 1:9 (w/w), about 0.01 to 3 % (w/w) of a surfactant, optionally about 0.1 to 5 % (w/w) of a preservative, about 1 to 150 mM of a buffer preferably citrate or histidine, a non aggregating agent such as arginine or lysine at a concentration range of 80 to 200 mM. Optionally, the composition may also comprise one or two tonicity modifiers such as NaCl, KCl at a concentration of about 0 to 150 mM for NaCl and KCI and/or bulking agents such as trehalose, mannitol, sorbitol or sucrose between 1 to 10 % (w/w) of mannitol, sorbitol, trehalose or sucrose.

Furthermore, the invention relates to a process for the preparation of pharmaceutical composition comprising a combination of IGF-1 and GH.

In the pharmaceutical formulations according to the present invention, the human growth hormone and insulin-like growth factor are preferably produced by recombinant means.

In a further embodiment both IGF-1 and GH, preferably in a composition according to the present invention can be administered to the patient, each in effective amounts or each in amounts that are sub-optimal but when combined are effective. Preferably such amounts are about 25 to 250 micrograms IGF-1/kg body weight/day and about 0.05 to 0.5 mg GH/kg body weight /week.

Preferably, the administration of the pharmaceutical formulation is by injection the injection is preferably parenteral such as via the subcutaneous, intramuscular, intravenous or infusion route, the pharmaceutical composition will be used most preferably as daily bolus injection and is preferably an immediate release formulation (IRF).

The patient to be treated is preferably a mammal, in particular human being but it may also be an animal.

In a further embodiment, the invention provides the use of the composition in the manufacture of a medicament for the treatment of a disease characterized by an increase in or control of, the amount of growth hormone in the plasma.

In particular, the invention provides a methods and compositions for the treatment of growth hormone deficiency (GDH); Turner Syndrome, Prader-Willi syndrome (PWS); short stature in children born with very low birth weight (VLBW), GDH in adults. Also for endocrine disorder for instance comprising administering to a patient suffering from a metabolic disorder characterized by partial endogenous growth hormone activity or signalling an amount of insuline-like growth factor-1 (IGF-1) and an amount of growth hormone (GH) that are effective in combination therapy to improve a metabolic abnormality in the patient. Wherein the patient has adult idiopathic short stature (ISS) syndrome, wherein the patient receives IGF-1 in a single administration per day and receives GH in a single administration per day, and wherein the patient receives the administration of IGF-1 and GH contemporaneously.

The invention also provides methods and compositions for children suffering from growth disorders characterized by partial endogenous growth hormone activity or signalling conditions. These growths which cause disorders in childhood persist into adulthood, and the affected adult can suffer from a variety of metabolic disorders.

According to the present invention hGH and hIGF-1 are used as a medicament or as pharmaceutical composition.

A valuable advantage of the present invention is to provide compositions which may be used as prefilled into a container such as syringes or ready to use formulations.

### Example 1

### Solubility tests

Mixtures of Increlex^{®} (10 mg/ml solution, formulated in 50 mM Acetate buffer at pH 5.4) and Nutropin AQ^{®} (5 mg/ml solution, formulated in 10 mM citrate buffer at pH 6) were prepared at volume ratios ranging from 9:1 to 1:9. The mixtures showed varying degrees of visible precipitation immediately or within a few hours of mixing. Mass spectroscopic analysis of precipitates formed in the Nutropin AQ^{®} and Increlex^{®} mixtures revealed the presence of both proteins in the precipitates. In table 1 are gathered observations and results relating to the clarity of co-mixtures prepared from commercialized products of IGF-1 (Increlex^{®}) and GH (Nutropin AQ^{®})

**Table 1**

| | | | **Observations** | | |
|---|---|---|---|---|---|
| **Ratio (v:v)** | **Increlex (mL)** | **Nutropin AQ (mL)** | **Initial (20MAR08)** | **24 hour (21MAR08)** | **1 week (27MAR08)** |
| **9:1** | 3.6 | 0.4 | Very slight free-floating precipitate pH=5.42 | Very slight free-floating precipitate | Very slight free-floating precipitate |
| **5:1** | 3.6 | 0.72 | Slight free-floating precipitate pH=5.51 | Slight free-floating precipitate | Slight free-floating precipitate |
| **2:1** | 3.6 | 1.8 | Free-floating precipitate pH=5.57 | Free-floating precipitate | Free-floating precipitate |
| **1:1** | 2.0 | 2.0 | Free-floating precipitate pH=5.64 | Free-floating precipitate | Clear with gelatinous film on glass |
| **1:2** | 1.8 | 3.6 | Turbid, heavy precipitate pH=5.74 | Clear with gelatinous film on glass | Clear with gelatinous film on glass |
| **1:5** | 0.72 | 3.6 | Free-floating precipitate pH=5.85 | Heavy, chunky precipitate | Clear with gelatinous film on glass |
| **1:9** | 0.40 | 3.6 | Free-floating precipitate pH=5.94 | Free-gloating precipitate | Free-floating precipitate |

The solubility of IGF-1 was confirmed to be greater that 20 mg/ml throughout the pH range of the mixtures (5.4-5.9), indicating that the IGF-1 solubility is not causative to the observed precipitate is not IGF-1. The solubility of GH in citrate, acetate or histidine buffers in the pH range was found to be buffer dependent. The results demonstrate a steep decline in solubility of acetate buffered solutions of GH at pH values below 5.6, which may contribute to the observed precipitation in the mixtures which result in the solutions.

However, mixtures of Nutropin AQ® with Increlex® placebo (which does not contain IGF-1, but is otherwise identical in composition to Increlex®), or mixtures of Increlex® with the Nutropin AQ® placebo, (which does not contain GH, but is otherwise identical in composition to Nutropin AQ®) remain clear in comparison, indicating that the reduced solubility of the proteins may also be related to interaction between the two proteins. Furthermore, Increlex®) diluted with Increlex® placebo to a final concentration of 2.5 mg/mL may be mixed with Nutropin AQ® in IGF-1:hGH ratios of 2.2:1 or greater with no precipitation, indicating that the interaction between the proteins is reversible.

### Example 2

### Comparison and preparation of co-mix compositions buffered in citrate at various pH

Lyophilized hGH was dissolved in a 10 mM citrate buffer at pH 6, containing 150 mM sodium chloride and 0.2% polysorbate 20, to a final concentration of 5 mg/ml. Solutions of IGF-1 in the different formulation buffers shown in Column 1 of Table 1 were prepared either by dialysis of the IGF-1 into the respective buffer or by reconstitution of lyophilized IGF-1 into the buffer. The final concentration of the IGF-1 solutions prior to mixing with the GH solutions was 10 mg/ml. The GH and IGF-1 solutions were mixed together in the various ratios shown in Table 2.

Visual appearance of co-mixtures prepared from GH in a citrate buffer with IGF-1 in various buffers at pH 5.4 and 6 are gathered in Table 2.

**Table 2**

| **IGF-1 formulation** | **Mixing ratio hGH:IGF-1** | **Visual appearance of mixtures after 1-2 Weeks at 5C** |
|---|---|---|
| 10 mM Citrate, pH 5.4 | 1:9 | Clear |
| | 3:7 | Clear |
| | 1:1 | Some particulates |
| | 7:3 | Particulates |
| | 9:1 | Cloudy Solution |
| 20 mM Citrate, pH 6 | 1:9 | Clear |
| | 3:7 | Slight particulates |
| | 1:1 | Slight particulates |
| | 7:3 | Slight particulates |
| | 9:1 | Slight particulates on mixing |
| 50 mM Citrate, pH 5.4 | 1:9 | Clear |
| | 3:7 | Clear |
| | 1:1 | Slight particulates |
| | 7:3 | Some particulates |
| | 9:1 | Cloudy Solution |
| 10 mM Acetate, pH 5.4 | 1:9 | Clear |
| | 3:7 | Slightly cloudy, clear after mixing |
| | 1:1 | Slightly cloudy solution |
| | 7:3 | Cloudy solution |
| | 9:1 | Cloudy solution |
| 50 mM Acetate, pH 5.4 | 1:9 | Clear |
| | 3:7 | Very slight particulates |
| | 1:1 | Particulates |
| | 7:3 | Cloudy suspension |
| | 9:1 | Cloudy suspension |
| 10 mM Phosphate, pH 6 | 1:9 | Clear after mixing |
| | 3:7 | Cloudy solution |
| | 1:1 | Cloudy solution |
| | 7:3 | Cloudy solution |
| | 9:1 | Cloudy solution |
| 50 mM Phosphate, pH 6 | 1:9 | Slightly cloudy, clear after mixing |
| | 3:7 | Cloudy after mixing |
| | 1:1 | Particulates |
| | 7:3 | Cloudy suspension |
| | 9:1 | Cloudy suspension |
| 10 mM Histidine, pH 5.4 | 1:9 | Clear |
| | 3:7 | Slightly cloudy after mixing |
| | 1:1 | Slightly cloudy after mixing |
| | 7:3 | Cloudy solution |
| | 9:1 | Cloudy solution |
| 50 mM Histidine, pH 5.4 | 1:9 | Clear |
| | 3:7 | Mostly clear on mixing |
| | 1:1 | Particulates |
| | 7:3 | Cloudy suspension |
| | 9:1 | Cloudy suspension |
| 10 mM Histidine, pH 6 | 1:9 | Clear |
| | 3:7 | Clear |
| | 1:1 | Slightly cloudy |
| | 7:3 | Slightly cloudy after mixing |
| | 9:1 | Cloudy solution |
| 50 mM Histidine, pH 6 | 1:9 | Clear |
| | 3:7 | Cloudy solution |
| | 1:1 | Cloudy solution |
| | 7:3 | Cloudy solution |
| | 9:1 | Cloudy solution |

The observations recorded in Table 2 show that solutions of IGF-1 in various buffers produce precipitation when mixed with GH formulated in citrate buffer at pH 6.

### Example 3

### Preparation of compositions and tests of clarity of citrate buffered composition

Approximately 19 mg/ml solutions of each protein (IGF-1 and hGH) were separately dialyzed into a 10 mM citrate buffer at pH 6.0, containing 10 mM arginine. Following overnight dialysis, the solution concentrations were determined by measurement of the ultraviolet (UV) absorbance at 280 nm. The final concentrations of the IGF-1 and hGH solutions were 14 and 21 mg/ml, respectively. Individual aliquots of each solution were constituted with the remaining excipients as shown in Table 3 and diluted to a final protein concentration of 10 mg/ml. Each pair of individually formulated protein solutions (IGF-1 and hGH) were mixed in a 1:1 ratio, to prepare mixtures containing 5 mg/ml of each protein. After the two protein mixtures were prepared, one of the two surfactants was added to both the individual protein formulations and co-mixtures, to the final concentrations. The solutions were inspected after 72 hours of refrigeration. The two mixtures which remained clear at this point (formulation compositions labelled A2 and A10 in Table 3) were stored in the refrigerator and inspected again to confirm that they remained clear after 70 days of storage. In Table 3 are gathered the results of appearance testing of citrate formulations after 72 hours at 5 °C.

**Table 3:**

| **Sample ID** | **Excipients** | **IGF-1 (10mg/ml)** | **hGH (10mg/ml)** | **Co-mix (5mg/ml IGF-1 + 5 mg/ml hGH)** |
|---|---|---|---|---|
| | | **Solution clarity after 72 hours** | | |
| A1 | 10 mM Arginine, 0.2% Polysorbate 20, 150 mM NaCl, 1% BzOH, 10 mM Citrate, pH 6.0 | Yes | No | No |
| **A2** | **100 mM Arginine, 0.2% Polysorbate 20, 50 mM NaCl, 1% BzOH, 10 mM Citrate, pH 6.0** | **Yes** | **Yes** | **Yes** |
| A3 | 10 mM Arginine, 0.2% Polysorbate 20, 150 mM NaCl, 0.25% Phenol, 10 mM Citrate, pH 6.0 | Yes | No | No |
| A4 | 10 mM Arginine, 0.3% Poloxamer 188, 150 mM NaCl, 1% BzOH, 10 mM Citrate, pH 6.0 | Yes | No | No |
| A5 | 100 mM Arginine, 0.3% Poloxamer 188, 150 mM NaCl, 1% BzOH, 10 mM Citrate, pH 6.0 | Yes | No | No |
| A6 | 10 mM Arginine, 0.03% Poloxamer 188, 150 mM NaCl, 1% BzOH, 10 mM Citrate, pH 6.0 | Yes | No | No |
| A7 | 100 mM Arginine, 0.03% Poloxamer 188, 150 mM NaCl, 1% BzOH, 10 mM Citrate, pH 6.0 | Yes | No | No i |
| A8 | 10 mM Arginine, 0.3% Poloxamer 188, 75 mM NaCl, 2.5% Mannitol, 1% BzOH, 10 mM Citrate, pH 6.0 | Yes | No | No |
| A9 | 10 mM Arginine, 0.3% Poloxamer 188, 5% Mannitol, 1% BzOH, 10 mM Citrate, pH 6.0 | Yes | No | No |
| **A10** | **100 mM Arginine, 0.3% Poloxamer 188, 5% Mannitol, 1% BzOH, 10 mM Citrate, pH 6.0** | **Yes** | **Yes** | **Yes** |

Both of the clear formulations (A2 and A10) contained 100 mM added argininium ion, and little or no added sodium chloride. Two formulations, labeled A1 and A9 in Table 3, which were almost identical in composition to A2 and A10, respectively, but contained a lower amount of added argininium ion (10 mM), did not remain clear.

### Example 4

### Preparation and comparative clarity test of histidine buffered composition

A 19 mg/ml solution of IGF-1 was dialyzed into a 10 mM Histidine buffer at pH 5.6 containing 10 mM arginine. Following dialysis, the solution concentration was determined by measurement of the ultraviolet (UV) absorbance at 280 nm to be 18 mg/ml. The solution was constituted with appropriate amounts of additional arginine, benzyl alcohol, surfactant (Polysorbate 20 or Poloxamer 188), sodium chloride, and mannitol to prepare the formulation compositions labelled B1 through B8 in Table 4. Aliquots of the IGF-1 only formulations were used to reconstitute lyophilized growth hormone to prepare corresponding formulations containing 5 mg/ml of each protein. The appearance of the solutions was observed after refrigeration at 5 °C for 24 hours. All formulations showed some precipitation at this point, except for the three formulations labelled B3, B4 and B8 in Table 4. These formulations were stored in the refrigerator for a further 65 days, and remained clear at the end of that that time. In Table 4 is gathered histidine buffered formulations at pH 5.6 after 24 hours at 5 °C.

**Table 4**

| **Sample ID** | **Excipients** | **IGF-1 (5 mg/mL)** | **Co-mix (5mg/mL IGF-1 + 5 mg/mL hGH)** |
|---|---|---|---|
| | | **Solution clarity after 72 hours** | |
| B1 | 10 mM Arginine, 0.2% Polysorbate 20, 150 mM NaCl, 1% BzOH, 10 mM Histidine, pH 5.6 | Yes | No |
| B2 | 10 mM Arginine, 0.02% Polysorbate 20, 150 mM NaCl, 1% BzOH, 10 mM Histidine, pH 5.6 | Yes | No |
| **B3** | **100 mM Arginine, 0.02% Polysorbate 20, 50 mM NaCl, 1% BzOH, 10 mM Histidine, pH 5.6** | **Yes** | **Yes** |
| **B4** | **100 mM Arginine, 0.3% Poloxamer 188 20, 50 mM NaCl, 1% BzOH, 10 mM Histidine, pH 5.6** | **Yes** | **Yes** |
| B5 | 10 mM Arginine, 0.3% Poloxamer 188 20, 150 mM NaCl, 1% BzOH, 10 mM Histidine, pH 5.6 | Yes | No |
| B6 | 10 mM Arginine, 0.03% Poloxamer 188 20, 150 mM NaCl, 1% BzOH, 10 mM Histidine, pH 5.6 | Yes | No |
| B7 | 0.3% Poloxamer 188 20, 5% Mannitol, 1% BzOH, 10 mM Histidine, pH 5.6 | Yes | No |
| **B8** | **100 mM Arginine, 0.3% Poloxamer 188, 5% Mannitol, 1% BzOH, 10 mM Histidine, pH 5.6** | **Yes** | **Yes** |

All of the three formulation mixtures which remained clear contained 100 mM argininium ion. The formulation mixture labelled B7, which corresponds exactly to the composition of the B8 formulation in Table 4 but without the added arginine, showed precipitation when observed after 24 hours of refrigeration, while the B8 formulation remained clear. Similarly, the formulation mixture labelled as B4 in Table 4 remained clear after prolonged refrigeration, while the B2 formulation, which contained only 10 mM Arginine, and additional sodium chloride, did not remain clear.

### Example 5

### Preparation of composition histidine buffered at pH 6

Approximately 19 mg/ml solutions of each protein (IGF-1 and hGH) were separately dialyzed into a 10 mM histidine buffer at pH 6 which included 10 mM arginine. Following overnight dialysis, the solution concentrations were determined by measurement of the ultraviolet (UV) absorbance at 280 nm. The final concentrations of the IGF-1 and hGH solutions after dialysis were 11 and 21 mg/ml, respectively. The solutions were individually constituted with appropriate amounts of additional arginine, benzyl alcohol, surfactant (Polysorbate 20 or Poloxamer 188), sodium chloride, and mannitol to prepare the two formulation compositions labelled C1 and C2 in Table 5. The individual protein formulations were mixed in a 1:1 ratio to prepare the co-mixtures, and the appearance of all 6 solutions was observed after refrigeration at 5 °C for 72 hours. Both sets of growth hormone formulations and co-mixtures showed some precipitation, possibly due to the high salt concentration (150 mM) in these formulations. In table 5 are gathered histidine buffered formulation at pH 6 after 72 hours at 5°C.

**Table 5:**

| **Sample ID** | **Excipients** | **IGF-1 (10mg/ml)** | **hGH (10mg/ml)** | **Co-mix (5mg/ml IGF-1 + 5 mg/ml hGH)** |
|---|---|---|---|---|
| | | **Solution clarity after 72 hours** | | |
| C1 | 100 mM Arginine, 0.3% Poloxamer 188, 150 mM NaCl, 1% BzOH, 10 mM Histidine, pH 6.0 | Yes | No | No |
| C2 | 0.3% Poloxamer 188, 150 mM NaCl, 1% BzOH, 10 mM Histidine, pH 6.0 | Yes | No | No |

### Example 6

### Preparation and comparison of citrate and histidine buffered composition

Two formulations were prepared with each protein (IGF-1 and GH), at final protein concentrations of 20 mg/ml (IGF-1) and 6 mg/ml (GH), respectively:
Formulation 1 : 10mM Citrate, 0.2% Polysorbate 20, 1% Benzyl Alcohol, 100mM Arginine, 50 mM NaCl, pH 6.2
Formulation 2 : 10mM Histidine, 100mM Arginine, 0.3% Poloxamer 188, 1% Benzyl Alcohol, 50 mM NaCl, pH 5.8

The formulations were prepared by buffer exchange of each protein by tangential flow-filtration, into each of the two buffers (buffer 1 and buffer 2), to prepare four stock solutions at the concentrations shown in Table 6. In Table 6 is gathered the preparation of stock solutions for formulation.

**Table 6:**

| **Buffer System** | **Concentration of protein after buffer exchange(mg/ml)** | |
|---|---|---|
| | **IGF-1** | **GH** |
| **Buffer 1:** 10mM Citrate, 50mM NaCl, 100mM Arginine pH 6.0 | 38 mg/ml | 15 mg/ml |
| **Buffer 2:** 10mM Histidine, 50mM NaCl, 100mM Arginine pH 5.6 | 29 mg/ml | 14 mg/ml |

Additional buffer and surfactant stock solutions were added to each stock solution with gentle mixing, followed by addition of the appropriate amount of neat BzOH (benzyl alcohol to achieve the final composition of formulations 1 and 2 with each protein. The protein concentrations of the IGF-1 and hGH formulations were 20 mg/ml and 6 mg/ml, respectively. The four formulations (two IGF-1 formulations and two hGH formulations) were then diluted and/or mixed to achieve the final formulations and co-mixtures in Table 7. The solutions were then stored at 2-8°C until further dilution/vialing. All protein solutions were sterile filtered using PES membranes and then aliquoted into 3 ml glass vials. The vials were stoppered, crimp-sealed and stored for up to 8 weeks in the refrigerator. The appearance of each solution was evaluated at 2 week intervals for 8 weeks. At the end of 8 weeks, all 14 solutions were still clear and colorless. In table 7 are gathered the results of visual appearance of citrate and histidine formulations containing 100 mM arginine.

**Table7**

| **Citrate Formulations: 10mM Citrate, 0.2% Polysorbate 20, 1% Benzyl Alcohol, 100mM Arginine, 50 mM NaCl, pH 6.2** | | | | | |
|---|---|---|---|---|---|
| **ID** | **Ratio** | **hGH** | **IGF-1** | **Solution appearance** | |
| | **GH:IGF-1** | **(mg/ml)** | **(mg/ml)** | **Immediately after mixing** | **After 8 weeks at 5 C** |
| **CA1** | 1:1.1 | 3 | 3.3 | Clear, Colorless Solution | Clear, Colorless Solution |
| **CA2** | 1:4 | 2.5 | 10 | Clear, Colorless Solution | Clear, Colorless Solution |
| **CA3** | GH only | 3 | - | Clear, Colorless Solution | Clear, Colorless Solution |
| **CA4** | IGF-1 only | - | 10 | Clear, Colorless Solution | Clear, Colorless Solution |
| **CC1** | 1:1.1 | 4.5 | 5 | Clear, Colorless Solution | Clear, Colorless Solution |
| **CC2** | GH only | 6 | - | Clear, Colorless Solution | Clear, Colorless Solution |
| **CC3** | IGF-1 only | - | 20 | Clear, Colorless Solution | Clear, Colorless Solution |

| **Histidine Formulations: 10mM Histidine, 100mM Arginine, 0.3% Poloxamer 188, 1% Benzyl Alcohol, 50 mM NaCl, pH 5.8** | | | | | |
|---|---|---|---|---|---|
| **ID** | **Ratio** | **hGH** | **IGF-1** | **Solution appearance** | |
| | **GH:IGF-1** | **(mg/ml)** | **(mg/ml)** | **Immediately after mixing** | **After 8 weeks at 5 °C** |
| **HB1** | 1:1.1 | 3 | 3.3 | Clear, Colorless Solution | Clear, Colorless Solution |
| **HB2** | 1:4 | 2.5 | 10 | Clear, Colorless Solution | Clear, Colorless Solution |
| **HB3** | GH only | 3 | - | Clear, Colorless Solution | Clear, Colorless Solution |
| **HB4** | IGF-1 only | - | 10 | Clear, Colorless Solution | Clear, Colorless Solution |
| **HD1** | 1:1.1 | 4.5 | 5 | Clear, Colorless Solution | Clear, Colorless Solution |
| **HD2** | GH only | 6 | - | Clear, Colorless Solution | Clear, Colorless Solution |
| **HD3** | IGF-1 only | - | 20 | Clear, Colorless Solution | Clear, Colorless Solution |

The chemical stability of the compositions over the eight week period was verified by periodic analysis of the formulations and co-mixes. They were stored refrigerated at 5 °C and at 25 °C, to detect the primary, stability-limiting, degradation products of IGF-1 (*des-*Gly,Pro-IGF-1) and GH (deamidated GH) and comparison of the degradation rates with established rates for the registered, long-term stable Increlex® (IGF-1, liquid for injection) and Nutropin AQ® (GH, liquid for injection) controls. The degradation rates are displayed in Tables 8 and 9. The formulations show no trend for the slow degradation of IGF-1 at 5 °C over the 8 week time period; however, new formulations and co-mixes stored at 25 °C show stability comparable to typical accelerated degradation rates observed for Increlex®. The deamidation of GH in the new formulations and co-mixes shows comparable rates with Nutropin AQ® controls, at both 5 °C and at 25 °C.

**Table 8**

| **Formulation** | **hGH (mg/mL** | **IGF-1 (mg/mL)** | **Excipients** | **Degradation rate (% increase in DGP-IGF-1 per week) at 25 C** |
|---|---|---|---|---|
| **CA1** | 3 | 3.3 | 10mM Citrate, 0.2% Polysorbate 20, 1% Benzyl Alcohol, 100mM Arginine, 50 mM NaCl, pH 6.2 | 1.04E-01 |
| **CA2** | 2.5 | 10 | | 1.05E-01 |
| **CA4** | | 10 | | 9.02E-02 |
| **CC1** | 4.5 | 5 | | 8.49E-02 |
| **CC3** | | 20 | | 8.96E-02 |
| **HB1** | 3 | 3.3 | 10mM Histidine, 100mM Arginine, 0.3% Poloxamer 188, 1% Benzyl Alcohol, 50 mM NaCl, pH 5.8 | 8.17E-02 |
| **HB2** | 2.5 | 10 | | 8.72E-02 |
| **HB4** | | 10 | | 7.31E-02 |
| **HD1** | 4.5 | 5 | | 7.96E-02 |
| **HD3** | | 20 | | 6.78E-02 |
| **Increlex*** | | 10 | 50 mM Acetate, 0.2% Polysorbate 20, 0.9% Benzyl Alcohol, 100 mM NaCl, pH 5.4 | 1.19E-01 |

| | | | | |
|---|---|---|---|---|
| * **Average degradation rate from 15 lots** | | | | |

**Table 9**

| **Formulation** | **hGH (mg/mL** | **IGF-1 (mg/mL)** | **Excipients** | **Deamidation rate (% increase per week)** | |
|---|---|---|---|---|---|
| | | | | **at 5 C** | **art 25 C** |
| **CA1** | 3 | 3.3 | 10mM Citrate, 0.2% Polysorbate 20, 1% Benzyl Alcohol, 100mM Arginine, 50 mM NaCl, pH 6.2 | 1.91E-02 | 2.85E-01 |
| **CA2** | 2.5 | 10 | | 2.09E-02 | 3.25E-01 |
| **CA3** | 3 | | | 1.56E-02 | 2.71E-01 |
| **CC1** | 4.5 | 5 | | 2.48E-02 | 3.04E-01 |
| **CC2** | 6 | | | 2.36E-02 | 3.00E-01 |
| **HB1** | 3 | 3.3 | 10mM Histidine, 100mM Arginine, 0.3% Poloxamer 188, 1% Benzyl Alcohol, 50 mM NaCl, pH 5.8 | 1.53E-02 | 1.33E-01 |
| **HB2** | 2.5 | 10 | | 2.01E-02 | 1.62E-01 |
| **HB3** | 3 | | | 1.43E-02 | 1.22E-01 |
| **HD1** | 4.5 | 5 | | 1.66E-02 | 1.51E-01 |
| **HD2** | 6 | | | 1.99E-02 | 1.44E-01 |
| **Nutropin AQ** | 5 | | 10mM Citrate, 0.2% Polysorbate 20, 0.25% Phenol, 150 mM NaCl, pH 6.0 | 2.12E-02 | 2.99E-01 |

### Example 7

### Preparation and comparison of histidine buffered compositions of IGF-1

Two formulations were prepared with IGF-1 at a final protein concentrations of 20 mg/ml (IGF-1):
- Formulation 1:: 20mM Histidine, 0.2% Polysorbate 20, 1% Benzyl Alcohol, 150mM Arginine, pH 5.8
- Formulation 2:: 50mM Histidine, 0.2% Polysorbate 20, 1% Benzyl Alcohol, 150mM Arginine, pH 5.8

The formulations were prepared by buffer exchange of protein into each of the two buffers, formulated by addition of surfactant and preservative, and evaluated for stability alongside Increlex® controls. The stability data at 5°C, 25°C and 40°C presented in Table 10.

**Table 10**

| **Peak/Group** | **Sample** | **Degradation rate (Day-1)** | | |
|---|---|---|---|---|
| | | **5C** | **25 C** | **40 C** |
| **% Active 1** | **50 mM Histidine formulation Increlex Control** | 1.43E-02 | 4.00E-02 | 1.30E-01 |
| | | 1.86E-02 | 3.71E-02 | 1.43E-01 |
| **% Main Peak** | **50 mM Histidine formulation Increlex Control** | -2.14E-02 | -5.90E-02 | -2.43E-01 |
| | | -2.90E-03 | -3.29E-02 | -2.10E-01 |
| **% Active 1** | **20 mM Histidine formulation Increlex Control** | 1.72E-04 | 1.87E-02 | 1.46E-01 |
| | | 2.67E-03 | 1.66E-02 | 1.39E-01 |
| **% Main Peak** | **20 mM Histidine formulation Increlex Control** | -1.36E-02 | -5.87E-02 | -3.03E-01 |
| | | 4.39E-03 | -1.75E-02 | -2.12E-01 |

### Example 8

### Stabilities data on combo formulations

**Table 11**

| **Temp** | **Months** | **Test/ Pull Date¹** | **Actual Months** | **Increlex (% Monomer)** | | **rhIGF-1 Only (% Monomer)** | | **Nutropin (% Monomer)** | | **rhGH Only (% Monomer)** | | **1:2.2 Co-form. (% Monomer)** | | **1:6.6 Co-form. (% Monomer)** | |
|---|---|---|---|---|---|---|---|---|---|---|---|---|---|---|---|
| | | | | **IGF-1** | **GH** | **IGF-1** | **GH** | **IGF-1** | **GH** | **IGF-1** | **GH** | **IGF-1** | **GH** | **IGF-1** | **GH** |
| n/a | 0 | 22Oct09 | 0.000 | 99.5 | -- | 99.7 | -- | -- | 99.9 | -- | 100.0 | 99.5 | 100.0 | 99.6 | 100.0 |
| 5°C | 3 | 27Jan10 | 3.189 | 99.3 | -- | 99.5 | -- | -- | 99.6 | -- | 99.9 | 99.5 | 100.0 | 99.6 | 100.0 |
| | 6 | 03May10 | 6.345 | 99.9 | -- | 99.9 | -- | -- | 99.5 | -- | 100.0 | 100.0 | 100.0 | 100.0 | 100.0 |
| 15°C | I | 18Nov09 | 0.888 | 99.6 | -- | NT | NT | -- | 99.5 | NT | NT | 99.4 | 100.0 | 99.5 | 100.0 |
| | 3 | 18Jan10 | 2.893 | 99.4 | -- | NT | NT | -- | 99.4 | -- | 99.8 | 99.4 | 99.9 | 99.4 | 100.0 |
| | 6 | 16Apr10 | 5.786 | 99.9 | -- | NT | NT | -- | 99.1 | -- | 99.9 | 100.0 | 100.0 | 100.0 | 100.0 |
| 25°C | 1 | 18Nov09 | 0.888 | 99.5 | -- | 99.5 | -- | -- | 99.7 | NT | NT | 99.2 | 100.0 | 99.3 | 100.0 |
| | 3 | 18Jan10 | 2.893 | 98.9 | -- | 98.8 | -- | -- | 98.5 | -- | 98.8 | 99.0 | 99.4 | 99.2 | 100.0 |
| | 6 | 16Apr10 | 5.786 | 99.2 | -- | 98.4 | -- | -- | 99.7 | NT | NT | 97.9 | 98.0 | 98.6 | 98.9 |
| 40°C | 1 | 18Nov09 | 0.888 | 97.7 | -- | 95.0 | -- | -- | 98.0 | -- | | 98.0 | 93.3 | 97.6 | 94.5 |
| | 3 | 18Jan10 | 2.893 | 92.3 | -- | 83.2 | -- | -- | 91.9 | -- | | 94.3 | 92.9 | 93.9 | 88.3 |

| | | | | | | | | | | | | | | | |
|---|---|---|---|---|---|---|---|---|---|---|---|---|---|---|---|
| ¹ Since samples are stores at 5°C until testing, the test date is used to trend 5°C data and the pull date is used to trend accelerated stability data for greatest accuracy. NT: Sample was not tested | | | | | | | | | | | | | | | |

These data have been produce by size exclusion chromatography and provide 1, 3 and 6 months stabilities.

### Example 9

### Establishment of an alternative co-formulation process of rhlGF-1 and rhGH

### 1. Materials and method

### 1.1. Raw materials

The following raw materials were used in the study and depicted in Table 12:

### 12 - Raw materials

| **Material** | **Supplier** |
|---|---|
| rhGH | Genentech |
| rhIGF-1 | Lonza |
| Citric acid | Sigma |
| Arginine-HCl | Merck |
| Phenol | Merck |
| Poloxamer 188 | BASF |
| Sucrose | Beghin Say |
| Sodium hydroxide | Merck |
| WFI | Cooper |
| Vials 5 mL VB type lyo | Schott |
| Stoppers 13 mm | West - CTSU |
| Crimps alu 13 mm | West |

### 1.2. Equipment

The following equipments were employed used for the study:
- Autoclave FEDEGARI,
- Bottles sterile polyethylene (PE) Nalgene ref. 2019,
- Cassettes BIOMAX PES ref. : PXB005A50 for diafiltration,
- Clean room, laminar flow hood,
- Cogent µScale MILLIPORE tangential flow filtration equipment,
- Filters MILLEX (33 mm) PES 0.22 µm (MILLIPORE),
- Filters MILLEX (33 mm) PVDF 0.22 µm (MILLIPORE),
- Glass beakers,
- Graduated cylinders,
- Magnetic stirrers,
- Micropipettes P1000,
- Oven FEDEGARI,
- Pharmacopoeia compliant vial observation equipment,
- Pump FLEXICON PF6 n°212118,
- Syringe PE 50 mL,
- Test tubes Eppendorf 1.5 mL,
- Test tubes Falcon 50 mL and 15 mL,
- Tygon tubes (1.6 mm + dedicated needle)
- Washing machine CORIMA.

### 2. Process and formula composition optimization

A process and formulation optimization trial study was performed. The formulation compositions were as described in Table 13 below:

### 13 - Composition of prototype formulations A3-c

| | | |
|---|---|---|
| **RATIO** | 2.2:1 | 2.2:1 |

| **NAME** | **A3** | **A3-c** |
|---|---|---|
| **Process strategy** | **A** | **A** |
| rhlGF-1 [mg/mL] | 7.9 | 7.9 |
| rhGH [mg/mL] | 3.6 | 3.6 |
| pH | 6.0 | 6.0 |
| Bulking agent [mM] | Sucrose 200 | Sucrose 140 |
| Arginine HCl [mM] | 150 | 150 |
| Histidine [mM] | - | - |
| Citrate [mM] | 20 | 20 |
| Succinate [mM] | - | - |
| Poloxamer 188 [mg/mL] | 2 | 2 |
| Polysorbate 20 mg/mL] | - | - |
| Benzyl alcohol [mg/mL] | - | - |
| Phenol [mg/mL] | 3.7 | 3.7 |
| Anti-oxidant [mM] | - | - |

### 2.1. rhIGF-1 tangential flow filtration (TFF) process

The rhIGF-1 TFF process was carried out with using the following parameters:
- IGF-1 quantity to engage :
   ∘ 80 mL of rhlGF-1 at 25 mg/mL,
- diafiltration concentration 25 mg/mL,
- exchange buffer: 20 mM citrate, 150 mM Arg. pH 6.0,
- Pellicon XL cassette: regenerated cellulose membrane, 5 KDa kDa cut-off,
- TMP 18-22 atm,
- Pump set at 12% of capacity,
- 6 diavolumes,
- rhIGF-1 final concentration 30 mg/mL.

### 2.2. Formulation of the proteins:

1. In this alternative process, all the excipients were first mixed together and then the protein was added to the excipients.
2. The solution of excipients was prepared at a pH lower than 6.0, so that the addition of the GH DS (at a pH around 7.5-8) would end up by giving a solution at pH lower than 7.

### 2.2.1. IGF-1 formulation

1. Weighed 8.44% of final volume of citrate 80 mM / arginine 600 pH 5.5 in a glass beaker.
2. Added 10% of final volume of phenol 3.7%.
3. Added 10% of final volume of poloxamer 188 2%.
4. Mixed to homogenization.
5. Added 3.84 g of sucrose.
6. Mixed to solubilization and homogenization.
7. pH value measured: 5.86.
8. While the solution was gently stirred, the volume corresponding to 1.6 g of diafiltered rhlGF-1 was added. The volume was calculated as follows:
9. 52.7 (mL) (Volume of diafiltered IGF-1) = 1600 [mg] (quantity of rhlGF-1 necessary) / 30.36 [mg/mL] (IGF-1 concentration)
10. Mixed to homogenization.
11. pH value measured: 6.05, therefore no pH correction considered.
12. Brought to final volume of 80 mL with WFI.
13. pH value measured: 6.05.
14. Filtration with 0.22 µm PES filters.
15. Filtration with 0.22 µm PVDF filters.

### 2.2.2. Formulation of GH

1. Added 25% of final volume of citrate 80 mM / arginine 600 pH 5.5 in a glass beaker.
2. Added 10% of final volume of phenol 3.7%.
3. Added 10% of final volume of poloxamer 188 2%.
4. Added 10% of final volume of WFI.
5. Mixed to homogenization.
   a. Limpid with few particles (probably of environmental origin),
   b. pH value measured: 5.9.
6. Added 4.83 g of sucrose.
7. Mixed to solubilization and homogenization.
   a. pH: 5.87;
8. Added 30 mL of GH DS while the solution is stirred gently.
9. Mixed to homogenization.
   a. pH value measured: 6.6.
   b. Final pH 6.0.
10. Filtration with 0.22 µm PES filters.
11. Filtration with 0.22 µm PVDF filters.

### 2.2.3 Co-formulation (or Co-mix)

To make 45 mL of final co-mix rhlGF-1/rhGH (2.2:1):
1. Added 17.9 mL of IGF-1 formulation to 27.1 mL of rhGH under magnetic stirring.
2. Mixed to homogenization.
3. Solution appeared limpid and without visible particles.
4. Filtration with 0.22 µm PES filters in the clean room of building 2: obtained limpid solution without visible particles / easy to withdraw carry out by syringe.

### 3. Application of the alternative process to a selected formulation using histidine as a buffer

### 3.1. Formulation feasibility

The formulation was carried out as described in § *2.2.1* for rhlGF-1 formulation, § *2.2.2* for rhGH formulation and § *2.2.3 for co-formulations 2.2:, 1 Co-mix* except for the use of:
- histidine instead of citrate as the buffering agent,
- pH 6.0 instead of 5.8,
- polysorbate-20 instead of poloxamer-188 as a surfactant,
- pure benzyl alcohol instead of a 10% phenol solution as a preservative,
- 2.5 % solution of HCl instead of 2.5% solution of citric acid to correct the pH,
- PVDF filters to filter the stand-alone protein products and the co-mix formulation,
- Co-formulations with ratios 1.1:1 and 6.6:1 were prepared instead of the co-formulation mix 2.2:1.

### 3.2 Optimization of the filtration-replicability

The process was carried out as described already in § 3.1 (in a lab at 21°C), except for the filtration process that was carried out according to the sequences below:.
- 150 mL of rhGH formulation subjected to a 1^{st} clarifying filtration with PES 0.22 µm + 2^{nd} sterilizing filtration with PVDF.
- 150 mL of rhGH formulation subjected to a 1^{st} clarifying filtration with PVDF 0.22 µm + 2^{nd} sterilizing filtration with PVDF.

The following observations were made:
- The rhGH solution before clarifying filtrations appeared as mildly opalescent and free of precipitates.
- After both filtration series, the rhGH formulations appeared free of particles.
- Visual inspections analyses confirmed the positive effect of using PVDF filters for final sterilizing filtration of formulations and co-formulations, i.e. the strong reduction of visible particles and the good stability when this type of filter is used.

**Table 14:**

| Formulation and co-formulation compositions | | | | | |
|---|---|---|---|---|---|
| | | **Compositions** | | | |
| | | Individual GH formulation | Individual IGF-1 formulation | Co-formulation Ratio IGF-1 / GH 1.1:1 | Co-formulation Ratio IGF-1 / GH 6.6:1 |
| pH | 5.8 | 5.8 | | | |
| Buffer | Histidine | 20 mM | | | |
| | Na bicarbonate | 7.5 mM | - | 5.6 mM | 2.5 mM |
| Surfactant | Polysorbate 20 | 0.2% | | | |
| Preservative | Benzyl alcohol | 1% | | | |
| Stabilizing agent | Arginine | 150 mM | | | |
| | | 6 mg/mL | 20 mg/mL | 5:4.5 (mg :mg)/ mL | 13.2 :2 (mg :mg)/ mL |

### Example 10

### Preparation processes for a rhlGF-1 and rhGH co-formulation

rhGH bulk (drug substance or DS) is a 20 mg/ml solution in bicarbonate buffer of concentration 7.5 mM. rhlGF-1 bulk (DS) is a 25-35 mg/ml solution in 200 mM citrate buffer.

3 different types of processes were prepared as follows (in the following designated process I, and alternative processes A and B):

### Steps of process I: :

- rhGH and rhlGF-1 diafiltration for buffer exchange (original DS buffer vs. Histidine (His) 20 mM / Arginine (Arg) 150 mM pH 5.8),
- for each individual protein, compounding was performed in the following order of introduction:
   - addition of exchange buffer to adjust concentration of DS,
   - addition of the solution of polysorbate PS 20,
   - addition of the solution of benzyl alcohol (BA),
- -co-mixing of individual formulations to produce a stable co-formulation.

### Steps of process A:

- rhGH and rhlGF-1 diafiltration for buffer exchange (original DS buffer vs. Histidine (His) 20 mM / Arginine (Arg) 150 mM pH 5.8),
- final compounding for each protein was carried out in the following order of introduction:
   - concentrated buffer,
   - solution of surfactant,
   - solution of preservative,
   - WFI (water for injection),
   - bulking agent (if any),
   - diafiltered drug substance,
   - pH correction with citric acid (or HCI),
   - WFI to final volume.
- co-mixing of individual formulations to produce a stable co-formulation.

### Steps of process B:

- rhIGF-1 diafiltration for buffer exchange (original DS buffer vs. Histidine (His) 20 mM / Arginine (Arg) 150 mM pH 5.8),
- no diafiltration of rhGH bulk, but direct formulation of rhGH bulk (i.e. addition of rhGH DS to excipients mixture without buffer exchange).
- Final compounding was carried out following the same order of introduction of components as in process A.
- co-mixing of individual formulations to produce a stable co-formulation.

## Claims

1. A pharmaceutical composition comprising IGF-1 and GH and
• a non-aggregating agent;
• a buffer;
• a non-ionic surfactant;
• a preservative; and
• a tonicity modifier or bulking agent
wherein
the non-aggregating agent is arginine present in the composition in a concentration ranging from 80 mM to 200 mM,
the buffer is selected from histidine or citrate at a concentration ranging from 1 to 50 mM, and
the tonicity modifier is sodium chloride at a concentration of 0 to 150 mM.

2. A pharmaceutical composition according to claim 1, wherein the non-aggregating agent is arginine at a concentration ranging from 100 mM to 150 mM.

3. A pharmaceutical composition according to any one of the preceding claims, wherein the buffer is at a concentration ranging from 10 to 20 mM.

4. A pharmaceutical composition according to any one of the preceding claims, wherein the surfactant is a non-ionic surfactant selected from a polysorbate (Tween) such as polysorbate 80, polysorbate 20 or a poloxamer such as poloxamer 188.

5. A pharmaceutical composition according to claim 4, wherein the non- ionic surfactant is polysorbate 20 at a concentration ranging from 0.1 to 0.3 % (w/w), preferably being 0.2 %(w/w).

6. A pharmaceutical composition according to claim 4, wherein the non-ionic surfactant is poloxamer 188 at a concentration ranging from 0.1 to 0.5% (w/w), preferably being 0.3%.

7. A pharmaceutical composition according to any one of the preceding claims, wherein the bulking agent is mannitol and/or sucrose.

8. A pharmaceutical composition according to claim 1, wherein the tonicity modifier is sodium chloride at a concentration ranging from 1 to 50 mM.

9. A pharmaceutical composition according to any one of the preceding claims, wherein the preservative is benzyl alcohol or phenol.

10. A pharmaceutical composition according to claim 8, wherein the preservative is benzyl alcohol at a concentration ranging from 0.2 to 2 % (w/w), preferably 1 %.

11. A pharmaceutical composition according to any one of the preceding claims, wherein the weight ratio of IGF-1:GH (w/w) ranges from 1:1 to 1:9 (w/w).

12. A pharmaceutical composition according to any one of claims 1 to 10, wherein the weight ratio of IGF-1:GH (w/w) ranges from 1:1 (w/w) to 7:1 (w/w) and is preferably at a value of 1.1:1 (w/w), 2.2:1 (w/w), 3.3:1 (w/w) or 6.6:1 (w/w).

13. A pharmaceutical composition according to any one of the preceding claims, wherein the pH of the pharmaceutical composition ranges from 5.0 to 6.5, preferably 5.4 to 6.2, and more preferably 5.8 to 6.2.

14. A pharmaceutical composition according to any one of claims 1 to 12 being a ready- to-use formulation in a pre-filled syringe or in a cartridge to be used in an injector device.

15. A process for the preparation of a pharmaceutical composition according to any one of the preceding claims, said process comprising:
a) Preparing a hGH solution in a buffer at a pH between 5 and 6.5 comprising the non-aggregating agent and the tonicity modifier or bulking agent;
b) Preparing a solution of IGF-1 by dialysing an IGF-1 preparation into the buffer used in step (a) comprising said non-aggregating agent and said tonicity modifier;
c) Adding the surfactant and the preservative to both stock solutions; and
d) Mixing together the solutions of hGH and IGF-1.

16. A process for the preparation of a pharmaceutical composition according to any one of claims 1 to 14, said process comprising:
a) Preparing a solution I by admixing the buffer, preferably histidine buffer, the non-aggregating agent, the surfactant, preferably polysorbate 20, the preservative, preferably benzyl alcohol, and optionally adjusting the volume with water, the solution I having or being adjusted to a pH of 5.8;
b) Preparing a solution of IGF-1, in the buffer and non-aggregating agent that are used in step (a), to obtain a solution II;
c) Adding solution II to solution I to obtain a solution III;
d) Preparing a solution IV by admixing the buffer, preferably histidine, the non-aggregating agent, the surfactant, preferably polysorbate 20, the preservative, preferably benzyl alcohol, and optionally adjusting the volume with water, the solution IV having or being adjusted to a pH of 5.8;
e) Preparing a solution of GH in the buffer and non-aggregating agent that are used in step (d), the GH optionally comprising sodium bicarbonate buffer, in order to obtain a solution V;
f) Adding solution V to solution IV to obtain a solution VI;
g) Optionally, independently filtering solutions III and VI;
h) Mixing filtered solutions III and VI at a ratio of IGF-1:GH (w/w) between 1:1 and 7:1 (w/w), preferably 1.1:1 (w/w), 3.3:1 (w/w) and 6.6:1, to obtain a solution VII; and
i) Optionally, filtering solution VII.

17. The process according to claim 16, wherein a liquid GH drug substance is directly mixed with solution IV without performing step (e).

18. The process according to claim 16, wherein the GH drug substance comprises a sodium bicarbonate buffer.

19. The process according to any one of claims 16 to 17, wherein the filtering steps are carried out on PVDF (polyvinylidene fluoride) filters.

20. The process according to any one of claims 16 to 18, wherein the non-aggregating agent is arginine at a concentration ranging from 100 mM to 150 mM.

## Patentansprüche

1. Eine pharmazeutische Zusammensetzung, umfassend IGF-1 und GH und
• ein aggregationsverhinderndes Mittel,
• einen Puffer,
• ein nichtionisches Tensid,
• ein Konservierungsmittel und
• ein Tonizitätsmodifizierungsmittel oder einen Füllstoff,
wobei
das aggregationsverhindernde Mittel Arginin ist, das in der Zusammensetzung in einer Konzentration im Bereich von 80 mM bis 200 mM vorliegt,
der Puffer ausgewählt ist aus Histidin oder Citrat in einer Konzentration im Bereich von 1 bis 50 mM, und
das Tonizitätsmodifizierungsmittel Natriumchlorid in einer Konzentration von 0 bis 150 mM ist.

2. Eine pharmazeutische Zusammensetzung gemäß Anspruch 1, wobei das aggregationsverhindernde Mittel Arginin in einer Konzentration im Bereich von 100 mM bis 150 mM ist.

3. Eine pharmazeutische Zusammensetzung gemäß einem der vorhergehenden Ansprüche, wobei der Puffer in einer Konzentration im Bereich von 10 bis 20 mM vorliegt.

4. Eine pharmazeutische Zusammensetzung gemäß einem der vorhergehenden Ansprüche, wobei das Tensid ein nichtionisches Tensid ist, ausgewählt aus einem Polysorbat (Tween), wie z.B. Polysorbat 80, Polysorbat 20, oder einem Poloxamer, wie z.B. Poloxamer 188.

5. Eine pharmazeutische Zusammensetzung gemäß Anspruch 4, wobei das nichtionische Tensid Polysorbat 20 in einer Konzentration im Bereich von 0,1 bis 0,3% (Gew./Gew.), vorzugsweise von 0,2% (Gew./Gew.), ist.

6. Eine pharmazeutische Zusammensetzung gemäß Anspruch 4, wobei das nichtionische Tensid Poloxamer 188 in einer Konzentration im Bereich von 0,1 bis 0,5% (Gew./Gew.), vorzugsweise 0,3%, ist.

7. Eine pharmazeutische Zusammensetzung gemäß einem der vorhergehenden Ansprüche, wobei der Füllstoff Mannit und/oder Saccharose ist.

8. Eine pharmazeutische Zusammensetzung gemäß Anspruch 1, wobei das Tonizitätsmodifizierungsmittel Natriumchlorid in einer Konzentration im Bereich von 1 bis 50 mM ist.

9. Eine pharmazeutische Zusammensetzung gemäß einem der vorhergehenden Ansprüche, wobei das Konservierungsmittel Benzylalkohol oder Phenol ist.

10. Eine pharmazeutische Zusammensetzung gemäß Anspruch 8, wobei das Konservierungsmittel Benzylalkohol in einer Konzentration im Bereich von 0,2 bis 2% (Gew./Gew.), vorzugsweise 1%, ist.

11. Eine pharmazeutische Zusammensetzung gemäß einem der vorhergehenden Ansprüche, wobei das Gewichtsverhältnis von IGF-1:GH (Gew./Gew.) von 1:1 bis 1:9 (Gew./Gew.) reicht.

12. Eine pharmazeutische Zusammensetzung gemäß einem der Ansprüche 1 bis 10, wobei das Gewichtsverhältnis von IGF-1:GH (Gew./Gew.) von 1:1 (Gew./Gew.) bis 7:1 (Gew./Gew.) reicht und vorzugsweise bei einem Wert von 1,1:1 (Gew./Gew.), 2,2:1 (Gew./Gew.), 3,3:1 (Gew./Gew.) oder 6,6:1 (Gew./Gew.) liegt.

13. Eine pharmazeutische Zusammensetzung gemäß einem der vorhergehenden Ansprüche, wobei der pH-Wert der pharmazeutischen Zusammensetzung von 5,0 bis 6,5, vorzugsweise von 5,4 bis 6,2, und besonders bevorzugt von 5,8 bis 6,2 reicht.

14. Eine pharmazeutische Zusammensetzung gemäß einem der Ansprüche 1 bis 12, die eine verwendungsfertige Formulierung in einer vorgefüllten Spritze oder in einer Patrone, die in einer Injektionsvorrichtung verwendet wird, ist.

15. Ein Verfahren zur Herstellung einer pharmazeutischen Zusammensetzung gemäß einem der vorhergehenden Ansprüche, wobei das Verfahren umfasst:
a) Herstellen einer hGH-Lösung in einem Puffer bei einem pH-Wert zwischen 5 und 6,5, umfassend das aggregationsverhindernde Mittel und das Tonizitätsmodifizierungsmittel oder den Füllstoff,
b) Herstellen einer IGF-1-Lösung durch Dialyse eines IGF-1-Präparats in den in Schritt (a) verwendeten Puffer, der das aggregationsverhindernde Mittel und das Tonizitätsmodifizierungsmittel umfasst,
c) Zugeben des Tensids und des Konservierungsmittels zu beiden Stammlösungen und
d) Vermischen der hGH- und IGF-1-Lösungen.

16. Ein Verfahren zur Herstellung einer pharmazeutischen Zusammensetzung gemäß einem der Ansprüche 1 bis 14, wobei das Verfahren umfasst:
a) Herstellen einer Lösung I durch Vermischen des Puffers, vorzugsweise Histidin-Puffer, des aggregationsverhindernden Mittels, des Tensids, vorzugweise Polysorbat 20, des Konservierungsmittels, vorzugsweise Benzylalkohol, und gegebenenfalls Einstellen des Volumens mit Wasser, wobei die Lösung I einen pH-Wert von 5,8 besitzt oder auf diesen eingestellt wird,
b) Herstellung einer Lösung von IGF-1 in dem Puffer und aggregationsverhindernden Mittel, die in Schritt (a) verwendet wurden, um eine Lösung II zu erhalten,
c) Zugeben von Lösung II zu Lösung I, um eine Lösung III zu erhalten,
d) Herstellen einer Lösung IV durch Vermischen des Puffers, vorzugsweise Histidin, des aggregationsverhindernden Mittels, des Tensids, vorzugsweise Polysorbat 20, des Konservierungsmittels, vorzugsweise Benzylalkohol, und gegebenenfalls Einstellen des Volumens mit Wasser, wobei die Lösung IV einen pH-Wert von 5,8 besitzt oder auf diesen eingestellt wird,
e) Herstellen einer Lösung von GH in dem Puffer und aggregationsverhindernden Mittel, die in Schritt (d) verwendet wurden, wobei das GH gegebenenfalls Natriumhydrogencarbonatpuffer umfasst, um eine Lösung V zu erhalten,
f) Zugeben von Lösung V zu Lösung IV, um eine Lösung VI zu erhalten,
g) gegebenenfalls unabhängiges Filtrieren der Lösungen III und VI,
h) Vermischen der filtrierten Lösungen III und VI in einem Verhältnis von IGF-1:GH (Gew./Gew.) zwischen 1:1 und 7:1 (Gew./Gew.), vorzugsweise 1,1:1 (Gew./Gew.), 3,3:1 (Gew./Gew.) und 6,6:1, um eine Lösung VII zu erhalten, und
i) gegebenenfalls Filtrieren der Lösung VII.

17. Das Verfahren gemäß Anspruch 16, wobei eine flüssige GH-Arzneistoffsubstanz direkt mit Lösung IV ohne Vorbildungsschritt (e) vermischt wird.

18. Das Verfahren gemäß Anspruch 16, wobei die GH-Arzneistoffsubstanz einen Natriumhydrogencarbonatpuffer umfasst.

19. Das Verfahren gemäß einem der Ansprüche 16 bis 17, wobei die Filtrierschritte auf PVDF (Polyvinylidenfluorid)-Filtern durchgeführt werden.

20. Das Verfahren gemäß einem der Ansprüche 16 bis 18, wobei das aggregationsverhindernde Mittel Arginin in einer Konzentration im Bereich von 100 mM bis 150 mM ist.

## Revendications

1. Composition pharmaceutique comprenant du IGF-1 et de la GH et
• un agent antiagrégant ;
• un tampon ;
• un surfactant non ionique ;
• un conservateur ; et
• un modificateur de tonicité ou un agent de charge,
dans laquelle
l'agent antiagrégant est de l'arginine présente dans la composition en une concentration comprise entre 80 mM et 200 mM,
le tampon est choisi parmi l'histidine ou le citrate en une concentration comprise entre 1 et 50 mM, et
le modificateur de tonicité est du chlorure de sodium en une concentration de 0 à 150 mM.

2. Composition pharmaceutique selon la revendication 1, dans laquelle l'agent antiagrégant est de l'arginine en une concentration comprise entre 100 mM et 150 mM.

3. Composition pharmaceutique selon l'une quelconque des revendications précédentes, dans laquelle le tampon est présent en une concentration comprise entre 10 et 20 mM.

4. Composition pharmaceutique selon l'une quelconque des revendications précédentes, dans laquelle le surfactant est un surfactant non ionique choisi parmi un polysorbate (Tween) tel que le polysorbate 80, le polysorbate 20 ou un poloxamère tel que le poloxamère 188.

5. Composition pharmaceutique selon la revendication 4, dans laquelle le surfactant non ionique est du polysorbate 20 en une concentration comprise entre 0,1 et 0,3 % (en poids), de préférence de 0,2 % (en poids).

6. Composition pharmaceutique selon la revendication 4, dans laquelle le surfactant non ionique est du poloxamère 188 en une concentration comprise entre 0,1 et 0,5 % (en poids), de préférence de 0,3 %.

7. Composition pharmaceutique selon l'une quelconque des revendications précédentes, dans laquelle l'agent de charge est du mannitol et/ou du sucrose.

8. Composition pharmaceutique selon la revendication 1, dans laquelle le modificateur de tonicité est du chlorure de sodium en une concentration comprise entre 1 et 50 mM.

9. Composition pharmaceutique selon l'une quelconque des revendications précédentes, dans laquelle le conservateur est de l'alcool benzylique ou benzylphénol.

10. Composition pharmaceutique selon la revendication 8, dans laquelle le conservateur est de l'alcool benzylique en une concentration comprise entre 0,2 et 2 % (en poids), de préférence de 1 %.

11. Composition pharmaceutique selon l'une quelconque des revendications précédentes, dans laquelle le rapport IGF-1 :GH (en poids) est compris entre 1:1 et 1:9 (en poids).

12. Composition pharmaceutique selon l'une quelconque des revendications 1 à 10, dans laquelle le rapport IGF-1:GH (en poids) est compris entre 1:1 (en poids) et 7:1 (en poids) et est de préférence de 1,1:1 (en poids), 2,2:1 (en poids), 3,3:1 (en poids) ou 6,6:1 (en poids).

13. Composition pharmaceutique selon l'une quelconque des revendications précédentes, dans laquelle le pH de la composition pharmaceutique est compris entre 5,0 et 6.5, de préférence entre 5,4 et 6,2, et plus préférablement entre 5,8 et 6,2.

14. Composition pharmaceutique selon l'une quelconque des revendications 1 à 12 qui est une formulation prête à l'emploi dans une seringue pré-remplie ou dans une cartouche à utiliser dans un dispositif d'injection.

15. Procédé pour la préparation d'une composition pharmaceutique selon l'une quelconque des revendications précédentes, ledit procédé comprenant :
a) la préparation d'une solution de hGH dans un tampon à un pH compris entre 5 et 6,5 comprenant l'agent antiagrégant et le modificateur de tonicité ou l'agent de charge ;
b) la préparation d'une solution de IGF-1 par dialyse d'une préparation de IGF-1 dans le tampon utilisé à l'étape (a) comprenant ledit agent antiagrégant et ledit modificateur de tonicité ;
c) l'ajout du surfactant et du conservateur aux deux solutions mères ; et
d) le mélange, ensemble, des solutions de hGH et de IGF-1.

16. Procédé pour la préparation d'une composition pharmaceutique selon l'une quelconque des revendications 1 à 14, ledit procédé comprenant :
a) la préparation d'une solution I en mélangeant le tampon, de préférence un tampon d'histidine, l'agent antiagrégant, le surfactant, de préférence du polysorbate 20, le conservateur, de préférence de l'alcool benzylique, et éventuellement en ajustant le volume avec de l'eau, la solution I ayant ou étant ajustée à un pH de 5,8 ;
b) la préparation d'une solution de IGF-1, dans le tampon et l'agent antiagrégant qui sont utilisés à l'étape (a), afin d'obtenir une solution II ;
c) l'ajout de la solution II à la solution I afin d'obtenir une solution III ;
d) la préparation d'une solution IV en mélangeant le tampon, de préférence de l'histidine, l'agent antiagrégant, le surfactant, de préférence du polysorbate 20, le conservateur, de préférence de l'alcool benzylique, et éventuellement en ajustant le volume avec de l'eau, la solution IV ayant ou étant ajustée à un pH de 5,8 ;
e) la préparation d'une solution de GH dans le tampon et l'agent antiagrégant qui sont utilisés à l'étape (d), la GH comprenant éventuellement un tampon de bicarbonate de sodium, afin d'obtenir une solution V ;
f) l'ajout de la solution V à la solution IV afin d'obtenir une solution VI ;
g) éventuellement, le filtrage indépendant des solutions III et VI ;
h) le mélange des solutions III et VI filtrées selon un rapport de IGF-1:GH (en poids) entre 1:1 et 7:1 (en poids), de préférence 1,1:1 (en poids), 3,3:1 (en poids) et 6,6:1, afin d'obtenir une solution VII ; et
i) éventuellement, le filtrage de la solution VII.

17. Procédé selon la revendication 16, dans lequel une substance médicamenteuse de GH liquide est directement mélangée à la solution IV sans réaliser l'étape (e).

18. Procédé selon la revendication 16, dans lequel la substance médicamenteuse de GH comprend un tampon de bicarbonate de sodium.

19. Procédé selon l'une quelconque des revendications 16 à 17, dans lequel les étapes de filtrage sont réalisées sur des filtres PVDF (fluorure de polyvinylidène).

20. Procédé selon l'une quelconque des revendications 16 à 18, dans lequel l'agent antiagrégant est de l'arginine en une concentration comprise entre 100 mM et 150 mM.
